# EUROPEAN PATENT SPECIFICATION

(11) **EP 3 804 662 B1**
(45) Date of publication and mention of the grant of the patent: **10.09.2025**
(21) Application number: 19820476.0
(22) Date of filing: 10.06.2019
(51) Int. Cl.: A61F 2/32, A61F 2/40, A61F 2/46, A61F 2/36

(54) **HIP OR SHOULDER PROSTHESIS AND POSITIONING INSTRUMENT**
HÜFT- ODER SCHULTERPROTHESE UND POSITIONIERINSTRUMENT
PROTHÈSE DE HANCHE OU D'ÉPAULE ET JEU D'INSTRUMENTS POUR LA MISE EN PLACE

(30) Priority: 11.06.2018 ES 201830565
(43) Date of publication of application: 14.04.2021
(73) Proprietor: Desarrollos Biomecánicos Innovasan S.L., 46010 Valencia (ES)
(72) Inventor: CORTES CUBERO, Javier, 46010 Valencia (ES); ATIENZA VICENTE, Carlos M., 46022 Valencia (ES); PEÑUELAS HERRAIZ, Andres, 46022 Valencia (ES); GARCIA TORRES, Fernando, 46022 Valencia (ES)
(74) Representative: Mora Granell, José Agustín
(86) International application number: PCT/ES2019/070399
(87) International publication number: WO 2019/238992

(56) References cited:
- EP-A2- 1 240 879
- EP-A2- 1 240 879
- EP-A2- 1 344 505
- EP-A2- 1 344 505
- WO-A1-2009/052294
- WO-A2-2011/028520
- US-A1- 2005 125 067
- US-A1- 2005 125 067
- US-A1- 2012 130 502
- US-A1- 2018 078 291
- US-A1- 2018 078 291

## Description

### FIELD OF THE INVENTION

The present invention relates to a hip or shoulder prosthesis (or arthroplasty) to replace the head of the femur or of the humerus in any case where required and which can be installed by a method that is relatively unaggressive for the patient.

It also relates to the instruments used for the positioning of the prosthesis, all in a minimally invasive manner.

### BACKGROUND OF THE INVENTION

In normal medical practice it is sometimes necessary to carry out a hip or shoulder replacement, in other words, to remove the head of the femur or of the humerus and position a device that restores the function of the joint, known as a hip or shoulder prosthesis or arthroplasty. This may be for different reasons, such as fractures, arthrosis, neoplasia, etc.

However, prosthesis positioning methods, whether for the hip or shoulder, are very aggressive and such surgical procedures involves very significant risks.

Any approach to replace hip or shoulder prosthesis (or arthroplasty), in any case where it is required, in order to minimize the aggressive nature of the surgery must overcome two important problems:
1. How can the femoral/humeral head be removed in a minimally invasive manner, reducing injury to the patient?
2. What type of device can be implanted easily and less aggressively once the femoral/humeral head has been removed?

For the second problem, in the case of the hip joint, the prostheses of patents US2003060889, US2016256281 and US2002095214 are known. These each comprise replacement prostheses for the acetabulum or head of the femur made up of smaller elements which are connected to one another in situ.

In the case of the shoulder joint, the removal of the head of the humerus poses similar, although somewhat different problems, due to the size of the joint and of its components.

EP1344505 is considered the closest prior art. It discloses a modular hip implant with a spherical component, a metaphyseal component attached to a bore of the spherical component, and a diaphyseal nail. The metaphyseal component is attached on top of the diaphyseal nail with a screw.

The applicant does not know of any prosthesis or tools that may be considered similar to the invention for solving these two initial questions.

### SUMMARY OF THE INVENTION

A prosthesis for replacing a femoral head or a humeral head according to the invention is defined in claim 1 and a kit for arthroplasty comprising said prosthesis is defined in claim 5.

The hip or shoulder prosthesis and the instruments described in their different embodiments overcome the problems of the prior art.

Throughout this description, the adjectives 'upper' and 'lower' should be considered as being used in a non-limiting way. Their use is intended to make the invention easier to understand without the need for complex explanations. Specifically, 'upper' should be considered to refer to the area of the element which, at the actual time of use, is closest to the patient's head. Conversely, 'lower' refers to the portion farthest from the patient's head.

The object of the hip or shoulder prosthesis is to reduce the complications caused by the required hip or shoulder replacement surgery in any case where positioning is necessary. For this reason, the design of the prosthesis prioritized the fact that its placement requires a surgical technique that is relatively unaggressive faced with the biomechanical requirements once implanted.

The design of the prosthesis and its instruments allows percutaneous positioning in a minimally invasive way, minimizing as much as possible the aggression of the surgery compared with standard prosthetic surgery.

In the case of the hip, for placement of the prosthesis, the same incisions are made as for a proximal femoral intramedullary nailing such as Gamma nail made by Stryker or PFNA nail made by Synthes. The prosthesis may also be positioned in the shoulder with two incisions.

The hip or shoulder prosthesis used to replace the femoral or humeral head includes the following components:
1. A spherical component (in the case of the hip prosthesis this replaces the head of the femur, and in the case of the shoulder it replaces the head of the humerus) of a substantially hemispherical shape. Said component has a rear face with a bore and recesses formed therein which facilitate its insertion in the patient's body.
2. A metaphyseal component is attached to the bore formed as a generally straight element with a transverse, approximately central, aperture. The metaphyseal component has a shank which is attached to the bore of the spherical component. Said component also has a longitudinal hole which passes through the aperture, the opening of said hole being on the side opposite the shank.
3. Thirdly, said prosthesis comprises a diaphyseal nail, which is intended for insertion in the femoral or humeral canal. This nail has a first segment where one or more fastening apertures for one or more distal screws for fastening to the femur or to the humerus are situated, and which can pass through the aperture of the metaphyseal component. It also has a second segment partly arranged in the aperture of the metaphyseal component.
4. Finally, said prosthesis has a locking device configured to be received in the hole and passing through the second segment of the diaphyseal nail.

In a first preferred embodiment, the metaphyseal component has grooving over its entire surface.

Preferably, the locking device passes completely through the diaphyseal nail and is threaded at its tip.

More preferably, the nail has a bracket in the second segment, above the metaphyseal component and resting thereon, on the opposite side of the shank.

The invention also relates to various instruments which assist in the positioning of the prosthesis.

Firstly, this relates to a cannulated extractor for the femoral or humeral head which comprises a tubular part with a grip at one end and a drill bit at the opposite end. The drill bit, which may be conical or cylindrical, allows a first Kirschner wire to pass tightly through its tip or center. Thus, the cannulated extractor can be guided to the femoral or humeral head without the tolerance of the passage affecting the result. For the Kirschner wire to pass through, the grip must have the corresponding aperture.

In a preferred embodiment, the drill bit is made up of various convergent and somewhat flexible elements. In that embodiment, the internal diameter of the tubular part is greater than that of the first Kirschner wire so that, if desired, an expander for the drill bit may be inserted which separates the convergent elements and helps lock the drill bit in the femoral or humeral head.

The invention also relates to a Kirschner wire centering device in the femoral or humeral diaphysis, neck, and head, which has the general shape of an inverted 'U.' This inverted 'U' is made up of two elements: a centering nail at one end and a handle-guide at the opposite end.
- The centering nail is intended for insertion in the femoral or humeral canal and has a vertical opening in its lower portion.
- The handle-guide, which remains outside the body once the centering nail has been inserted inside the femur or the humerus, also has a vertical opening in its lower portion, the upper edge of which is aligned with the upper edge of the vertical opening of the centering nail. This alignment may be by means of a sleeve arranged in the vertical opening of the handle-guide. That sleeve has an upper passage, for a first Kirschner wire, aligned with the upper edge of the vertical opening of the centering nail.

The vertical opening may therefore be straight or have a form complementary to that of the sleeve. The sleeve may have another passage for a second Kirschner wire.

The invention also relates to a spherical component insertion device which also has an inverted 'U' shape, with a handle with an open recess through the lower edge, and with an upper edge oriented towards the bore of the spherical component which is mounted on the insertion device. For this mounting, the spherical component insertion device has a support that is compatible with the recesses of the spherical component and can be released using an actuator that can be accessed from the outside (from the handle, for example).

In addition, the invention comprises an insertion device for the metaphyseal component, which consists of a straight, hollow rod, with a releasable clamp for the metaphyseal component at one end and a gripper at the opposite end.

Preferably, it comprises two tubular, coaxial bodies and the clamp is made up of protrusions, on the outer tubular body, of a bayonet closure for the corresponding clips of the metaphyseal component. It also comprises a thread close to the gripper for locking the relative movement between both tubular bodies.

Finally, the invention also relates to an insertion device for the diaphyseal nail, which can be used with the nail, and which has an inverted 'U' shape, with a hand grip with a groove open towards the lower portion thereof at a first end and a removable connection for the diaphyseal nail at the opposite end. This groove is provided to hold the insertion device for the metaphyseal component. The hand grip may be the same element as the handle-guide of the Kirschner wire centering device, changing only the centering nail through the removable connection. Further, it may also be the handle of the spherical component insertion device positioning the support for the spherical component.

The handle-guide of the centering device, the handle of the spherical component insertion device and the hand grip of the insertion device of the diaphyseal nail may each have sets of marks with a transparency to X-rays that is different from the rest of the centering device, with different development angles, in other words, which has a transverse depth that is oriented at different angles.

Other variants of the prosthesis and of the different instruments will be shown below.

### BRIEF DESCRIPTION OF THE DRAWINGS

For a better comprehension of the invention, the following drawings of a non-limiting embodiment are attached.
FIG. 1 is a perspective view of an example of a spherical component located in an insertion device of the appropriate spherical component.
FIG. 2 is a perspective view of an embodiment of the metaphyseal component near the spherical component.
FIG. 3 is a perspective view of the diaphyseal nail and the prosthesis mounted according to the embodiments of Figures 1 and 2.
FIG. 4 is a perspective view of the anterior spherical component insertion device.
FIG. 5 is a perspective view of a Kirschner wire centering device (centering nail + guide handle).
FIG. 6 is a perspective view of a Kirschner-wire centering device with an example of a sleeve placed on a patient's body (in this case on the hip), indicating the incisions to be made.
FIG. 7 is a perspective view of a first version of the femoral or humeral head extractor.
FIG. 8 is a perspective view of the insertion device of the metaphyseal component.
FIG. 9 illustrates details of the insertion device of the metaphyseal component.
FIG. 10 is a perspective view of the coupled spherical-component and metaphyseal-component insertion devices.
FIG. 11 is a perspective view of the diaphyseal nail together with an example of the diaphyseal nail insertion device.
FIG. 12 is a detail view of the orientation marks.
FIG. 13 is a view of the locking device.
FIG. 14 is a view of a second embodiment of the femoral or humeral head extractor.
FIG. 15 is a longitudinal section of the femoral or humeral head extractor of FIG. 14.

### DETAILED DESCRIPTION OF THE INVENTION

Next, an embodiment of the invention will be described briefly as a non-limiting illustrative example. It will be described with respect to a hip prosthesis, although as indicated, it is also applicable with respect to a shoulder prosthesis.

The prosthesis according to the invention is made up of three independent bodies, which in the embodiment shown in the figures correspond to:
- A spherical component **1** with the general shape of a hemisphere. It may be bipolar/biarticular or unipolar.
- A metaphyseal component **2,** for supporting the spherical component **1,** to which it is connected inside the patient's body. It is produced in different lengths to control the femoral offset.
- A diaphyseal nail **3,** which passes through an aperture **21** of the metaphyseal component **2** in order to be fastened in the appropriate position. The diaphyseal nail is produced with different diameters and lengths.

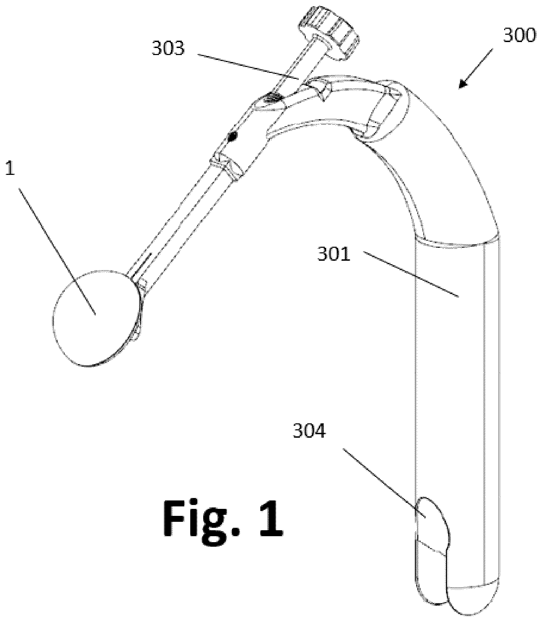

In FIG. 1 the spherical component **1** can be seen, which has a spherical face **11,** that may be formed to be substantially half of a sphere, although it may alternatively be formed to be somewhat more than half of a sphere, or somewhat less than a half sphere. It also has a rear face **12** for fastening to the metaphyseal component **2,** and therefore includes a bore **13,** which in the example shown is threaded. Ideally, the threading is conical. The rear face **12** of the spherical component **1** has recesses **14** for a first instrument which is the spherical component insertion device **300.** The spherical component **1** will be produced in different sizes so that the surgeon may select the most suitable.

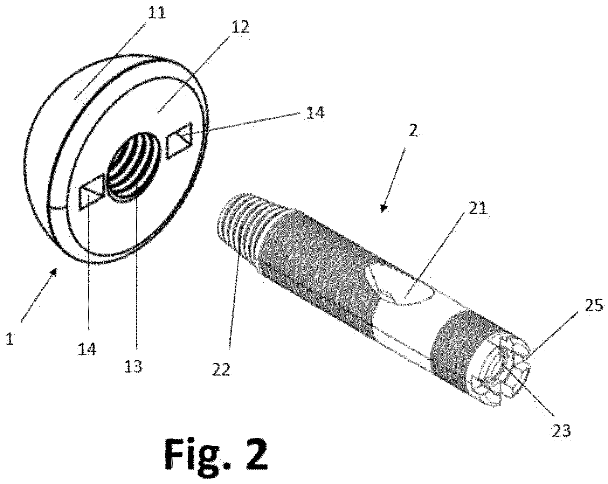

The metaphyseal component shown in **2** of FIG. 2 is connected to the spherical component **1** by means of a shank **22** which may be threaded or may include another type of fastener consistent with the bore **13.** Said component also has the transverse aperture **21** through which the diaphyseal nail **3** is inserted, which is positioned inside the femoral canal, and which allows both components **1, 2** to be immobilized. Said component also comprises a longitudinal hole **23** on the opposite side of the shank **22** where a locking device **24** is inserted to rigidly connect the metaphyseal component **2** to the diaphyseal nail **3.** The locking device **24** will usually be a threaded screw, the tip of which passes totally or partly through diaphyseal nail **3.** In the embodiments shown, the metaphyseal component **2** has a grooving over its entire surface to improve support in the femur. Other types of finish are also possible.

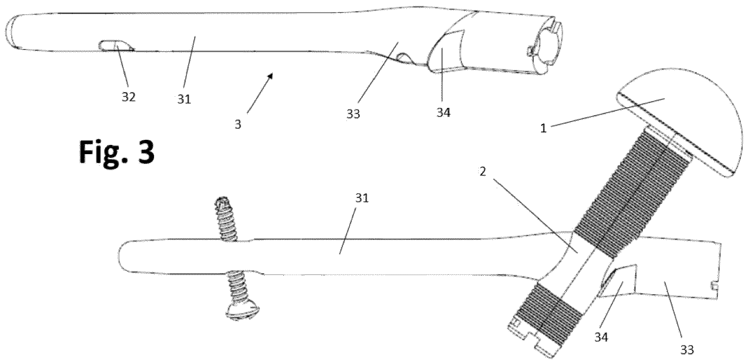

Finally, the diaphyseal nail **3** is configured for insertion in the femoral canal and has a first segment **31** with an increasing cross section, where one or more fastening apertures **32** configured for one or more screws for fastening to the femur. The second segment **33** of the diaphyseal nail **3** is configured to be engaged within the transverse aperture 21, the upper portion of which projects upwardly. Said diaphyseal nail **3** is therefore prepared to receive the locking device **24.** In FIG. 3 said diaphyseal nail **3** is shown with a support bracket **34** at the edge of the aperture **21** of the metaphyseal component **2,** which forms a stop when the diaphyseal nail **3** is inserted and which helps transmit the moments created with the movement of the joint and the load on walking when the patient rests on the operated leg.

The angle which is formed between the metaphyseal component **2** and the diaphyseal nail **3** (cervico-diaphyseal angle) will be approximately 125° (it may be larger or smaller, for example between 120° and 130°).

Next, the preferred method of positioning the hip prosthesis will be described, taking the opportunity to describe the necessary instruments. These instruments form part of the invention, as it is the most suitable way of positioning the elements of the prosthesis.

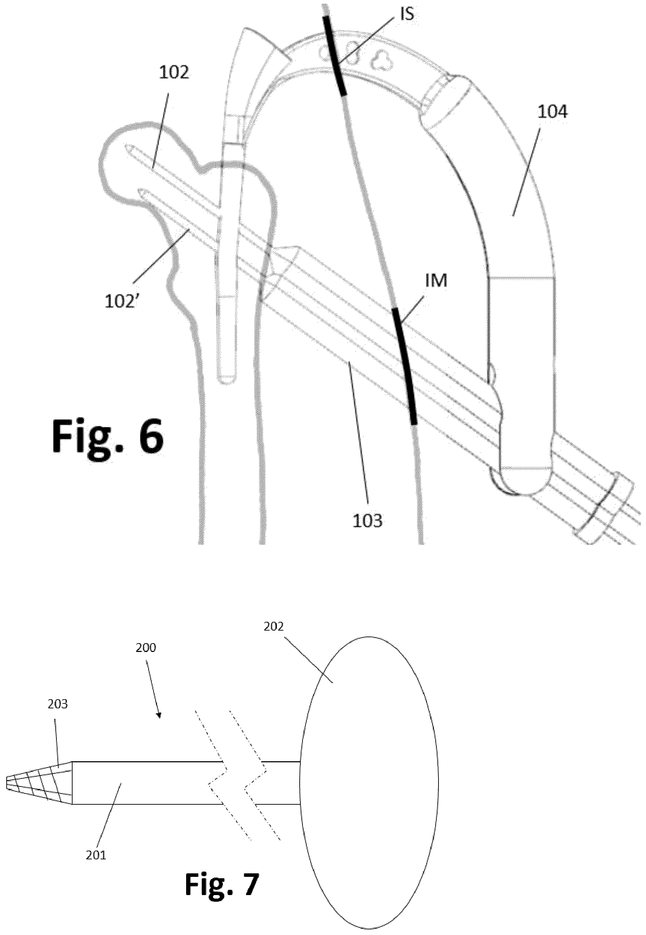

The prosthesis positioning method requires two or three incisions (FIG. 6):
- an upper incision **(IS)** slightly proximal to the greater trochanter;
- a middle incision **(IM)** which will be determined by the site through which the Kirschner wires enter in accordance with the Kirschner wire centering device;
a lower incision (not shown) which will be determined by the positioning of the distal screw/s. The lower incision may not be necessary as it may coincide with the middle incision **(IM).**

First, the femoral head is removed. This is carried out using a cannulated femoral or humeral head extractor **200,** which is inserted in the middle incision **(IM).** To remove the femoral head, the cannulated femoral head extractor **200** must penetrate in the most perpendicular and centered manner possible into the cancellous bone of the femoral head.

Accordingly, a first Kirschner wire **102** is used as a guide, being inserted through the middle incision **(IM).** A second Kirschner wire **102'** may also be applied through the middle incision **(IM),** as will be shown below.

This Kirschner wire, which serves as a guide for the cannulated femoral head extractor **200,** must be centered in the femoral diaphysis, neck, and head. The Kirschner wires **102, 102'** are locked in the correct position by means of a Kirschner wire centering device **100** having the general shape of an inverted 'U.'

The Kirschner wire centering device **100** has a Kirschner wire centering nail **101** at one end and a handle-guide **104** which forms the rest of the Kirschner wire centering device **100.** The centering nail **101** is intended for insertion in the femoral canal through the upper incision **IS,** whilst the handle-guide **104** is arranged outside the patient's body.

Prior to the insertion of the centering nail **101,** an entry will have been made through this upper incision **IS** in the tip of the greater trochanter with a drill bit of suitable diameter, and an opening will also have been made in the upper area of the articular capsule of the hip, which allows the femoral head to be removed later through this opening using the cannulated femoral head extractor **200.**

Both the centering nail **101** and the handle-guide **104** have vertical openings **105, 105'** on the lower portion thereof which allows the Kirschner wire centering device **100** to be removed once the Kirschner wires **102, 102'** have been positioned. The upper edges of both openings will be aligned.

The centered positioning of the first Kirschner wire **102** is vital, and the surgeon must therefore check its position using the image intensifier that is usually available in operating rooms or using another similar method.

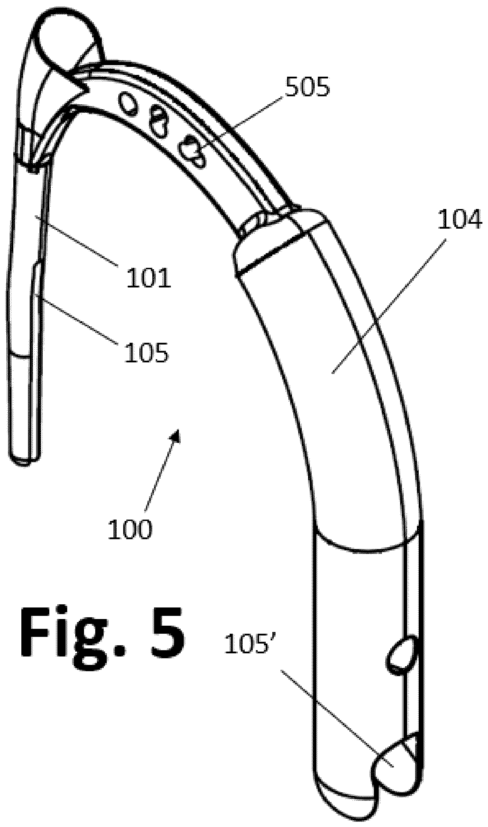

FIGS. 5 and 6 show the Kirschner wire centering device **100** arranged in the position of use, with the Kirschner wires **102, 102'** positioned. A sleeve **103** may be used which helps align the two Kirschner wires **102, 102',** and which can be removed from the handle-guide **104.** When the sleeve **103** is used, said sleeve will have an upper passage for the first Kirschner wire **102,** which will be that actually aligned with the upper edge of the vertical opening **105** of the centering nail **101.** The sleeve **103** may have more passages for more Kirschner wires **102, 102'.**

After being used for positioning the Kirschner wires **102, 102'** used, the Kirschner wire centering device **100** and the sleeve **103,** if used, are removed.

A preferred model of a cannulated extractor is shown in FIG. 7. It includes a tubular part **201,** in other words, cannulated, with a grip **202** at one end and a drill bit **203,** which may be conical or cylindrical, at the opposite end. The drill bit **203** allows the passage of the first Kirschner wire **102** tightly through its center. Thus, the cannulated extractor **200** can be guided to the femoral head without the tolerance of that passage affecting the result.

In one embodiment, the drill bit **203** may be formed of various internally convergent and somewhat flexible elements. In this embodiment, the internal diameter of the tubular part **201** is greater than that of the first Kirschner wire **102** so that, if desired, an expander (not shown) of the drill bit **203** can be inserted which separates the convergent elements and helps locking the drill bit **203** in the femoral head.

The second Kirschner wire **102'** will serve to anchor the femoral head to prevent the rotation thereof when the cannulated femoral head extractor **200** is inserted in the femoral head. It is therefore optional. In FIG. 6 said second Kirschner wire is shown situated beneath the first Kirschner wire **102,** but the exact position may vary.

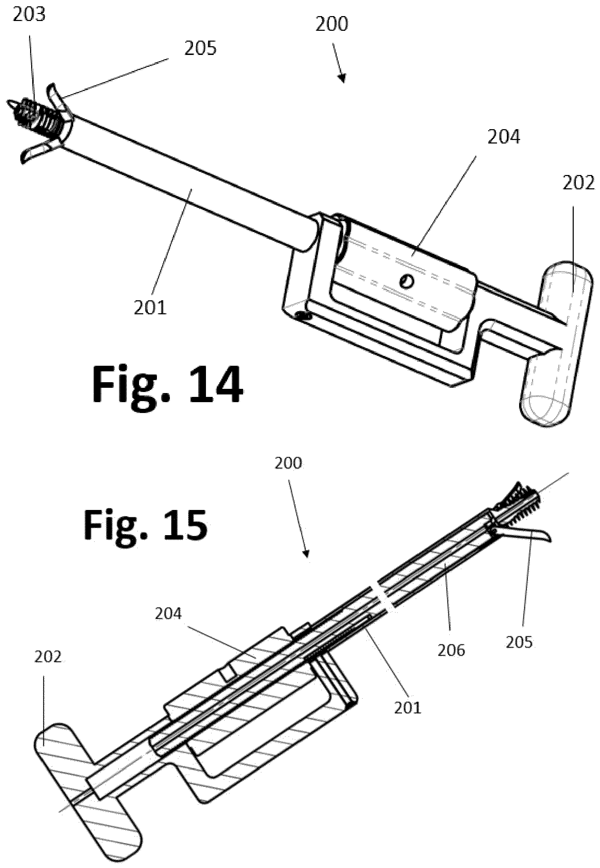

FIG. 14 shows a second example of a cannulated extractor **200** which also comprises the tubular part **201,** the grip **202** and the drill bit **203.** In addition, this solution has a holder **204** which acts on retractable claws **205** which project or are withdrawn close to the drill bit **203.** In the embodiment shown, the holder **204** is a nut on a screw **206** inside the tubular part **201,** which is also cannulated for the passage of the Kirschner wire **102.** The inner screw **206** is what pushes or retracts the retractable claws **205** as said screw is connected thereto, as shown in FIG. **15****.**

The tip of the retractable claws **205** is arranged to open on coming through the apertures provided for this purpose close to or in the drill bit **203.** For example, as shown, said tip may have a curved inner portion (oriented towards the drill bit) to form an inclined plane which initially helps said tip come out of the tubular part **201.**

The surgeon may therefore increase the grip on the femoral or humeral head once the drill bit is locked thereon, preventing relative rotation.

Before inserting the cannulated femoral head extractor **200** through the middle incision IM, the lateral cortical bone of the femur must have been drilled from the same middle incision using a drill bit of sufficient diameter to allow the cannulated extractor **200** to pass through the aperture that is thereby made. A cannulated drill bit, which will use the same Kirschner wire as a guide, will be used for this drilling.

The next step is to insert the cannulated femoral head extractor **200** from the middle incision MI to the femoral head. When reaching the cancellous bone of the femoral head, as the bone becomes harder on approaching the subchondral bone, greater grip is achieved with the rotations of the cannulated extractor **200.** The second wire prevents the rotation of the head within the acetabulum when inserting the cannulated extractor **200.**

Once the femoral head is secured to the extractor, the Kirschner wires **102, 102'** are removed and the affected limb is adducted. Then with the help of the cannulated femoral head extractor **200** the femoral head is dislocated by the upper portion of the joint (through the opening already made in the upper area of the articular capsule) for extraction through the upper incision **IS.** Once extracted, the cannulated extractor **200** can be removed through the middle incision **IM.**

Using this femoral head removal technique, the integrity of the articular capsule and of the acetabular labrum is preserved. This means that the hip retains its own stabilizing elements, providing the device with the correct stability and preventing dislocations. It also requires a smaller incision and less soft tissue dissection compared with any other prosthesis approach (hip arthroplasty), which therefore reduces bleeding during surgery.

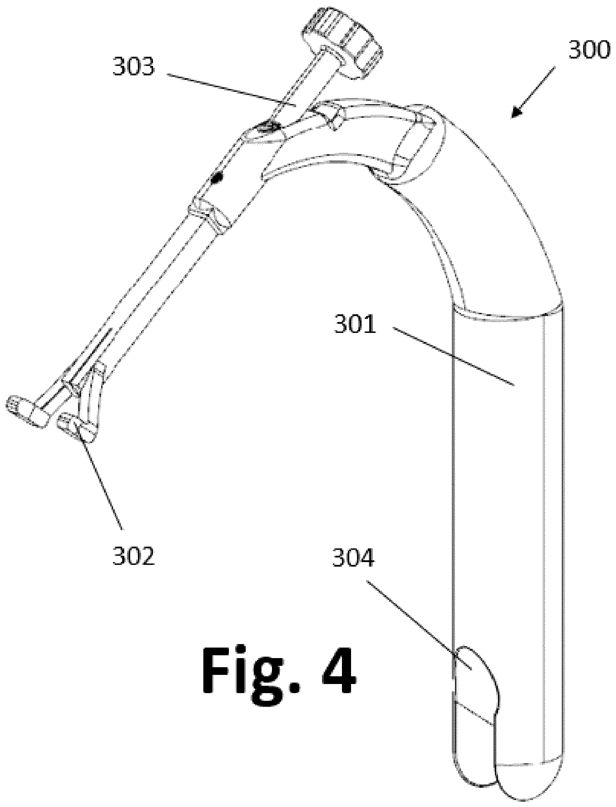

Next, the spherical component insertion device **300** is used, on which the spherical component 1 will be mounted. As can be seen in the embodiment shown in FIG. 1 and 4, said insertion device is somewhat similar to the Kirschner wire centering device **100,** in that said insertion device has a curved handle **301** and a support **302** for the spherical component **1.** This support **302** can be connected sufficiently rigidly to the recesses **14** of the spherical component **1,** and the recesses **14** can in turn be released when desired using an external actuator **303,** which in the case shown is a screw which separates the arms of the support **302.**

As can be seen, the handle **301** of the spherical component insertion device **300** has a recess **304** which is oriented towards the bore **13** of the spherical component **1.** In other words, any straight equipment which is inserted through the recess **304** will contact the bore **13** without the need for further operations. The lower portion of the recess **304** will be open to allow the handle **301** to be extracted regardless of the presence of an element positioned within the recess **304.**

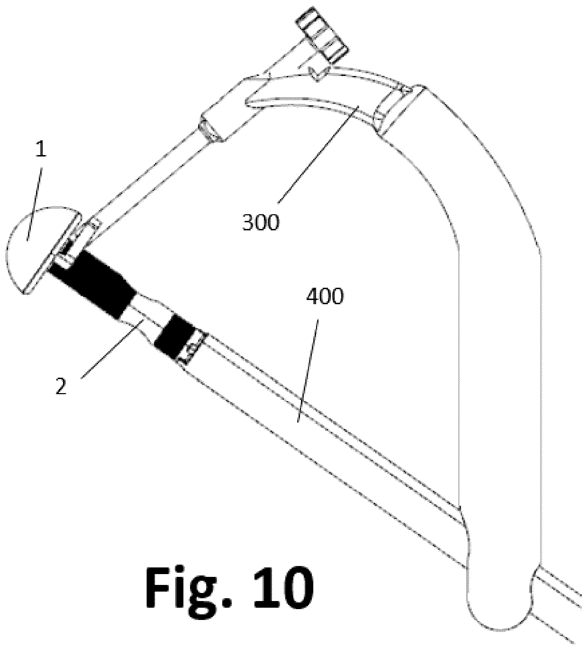

A metaphyseal component insertion device **400** can therefore be used, which consists of two hollow, elongated, coaxial rods **401, 402.** The outer, tubular rod **401** comprises a clamp **403** for the metaphyseal component **2** at one end and the inner rod **402** has a gripper **404** at the opposite end. The outer rod **401** will have an appropriate outer diameter for perfect alignment with the bore **13** when positioned in the recess **304** (FIG. 10). The surgeon will insert the metaphyseal component **2** through the middle incision **IM** and fasten said component to the bore **13** in a suitable manner, usually by threading. An indicator on the metaphyseal component insertion device **400** will indicate to the surgeon when said component is in the correct position for the aperture **21** to be perfectly prepared to receive the diaphyseal nail **3.**

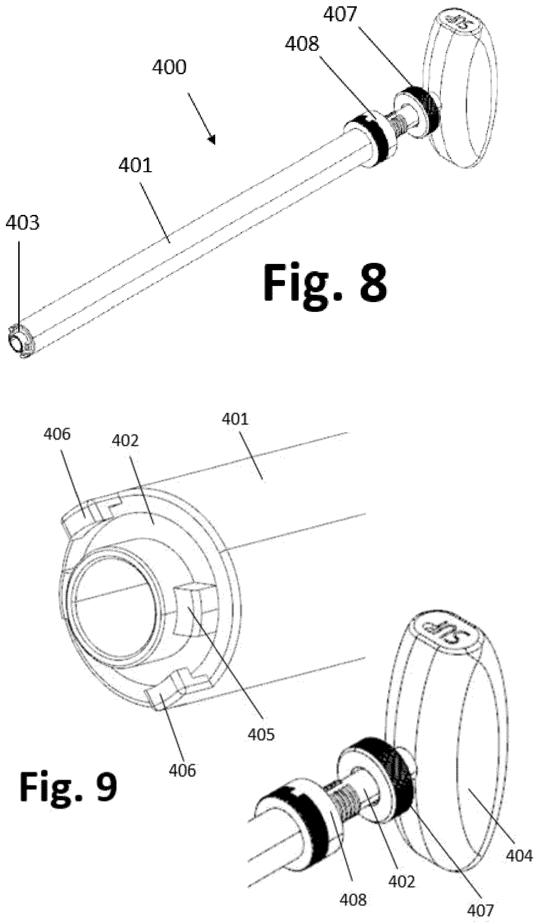

FIG. 9 shows two details of the preferred metaphyseal component insertion device **400.** The relative movement between both rods **401, 402** will allow the clamp **403** to be released or held it fixed. It can be seen that the clamp **403** is made up of protrusions **406** of a bayonet closure on the outer tubular body for the corresponding clips of the metaphyseal component **2.** Moreover, two projections **405** are arranged on the inner rod **402** which must enter the corresponding notches **25** of the metaphyseal component **2.** If both rods **401, 402** rotate together, the projections **405** ensure that the metaphyseal component **2** is threaded in the bore **13.** If said rods rotate independently, the projections **405** may cause the clamp **403** to disconnect in the bayonet configuration. A thread **407** close to the gripper **404** rigidly connects the movement between both coaxial rods **401, 402 on** tightening against the outer rod **401** a flange **408,** which can slide relative to the rods **401, 402,** but which cannot rotate relative to the inner rod **402.** Teeth, or any other method, will ensure that the closeness of the flange **408** prevents the outer rod **401** from rotating.

Once the spherical component **1** is fastened to the metaphyseal component **2,** the spherical component insertion device **300** can be removed, releasing the recesses **14** by means of the actuator **303.**

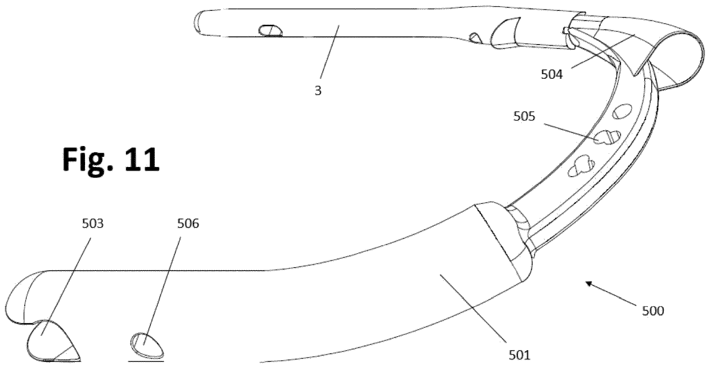

Next the diaphyseal nail **3** may be positioned using a diaphyseal nail insertion device **500,** which is shown in FIG. 11. Said diaphyseal nail insertion device **500** has a hand grip **501** which may be identical to the handle **301** of the spherical component insertion device **300.** It will have a groove **503** in its lower portion which can hold the metaphyseal component insertion device **400.** The diaphyseal nail **3** in any removable form is arranged at the other end of the diaphyseal nail insertion device **500.** Said diaphyseal nail insertion device **500** will have a release mechanism **504** of the diaphyseal nail **3** which can be accessed from the outside, for example, a screw which holds or releases the diaphyseal nail **3.**

The diaphyseal nail insertion device **500** may also be formed with an orifice **(506)** for the passage of the distal screw or of the corresponding tool. As can be seen in FIG. 6, the middle incision **IM** is used for the positioning thereof.

The diaphyseal nail **3** is then inserted through the upper incision **IS** until said nail passes through the aperture **21** of the metaphyseal component **2.** At that moment the diaphyseal nail **3** is locked because the size of its second segment **33** is related to that of the aperture **21,** particularly if the diaphyseal nail **3** has a bracket **34.**

Once the diaphyseal nail **3** is in position, the locking device **24** is inserted through the hole inside the inner rod **402** of the metaphyseal component insertion device **400,** and is suitably screwed or fastened. It is preferable that the locking device **24** terminates with a threaded area. The locking device **24** may be pre-installed in the metaphyseal component **2,** or in the metaphyseal component insertion device **400** so that fastening only requires the introduction of the fastening or screwing tool.

Once the locking device **24** is placed, the metaphyseal component is disconnected from the metaphyseal component insertion device. At that moment the anteversion of the hip arthroplasty device relative to the femur is selected.

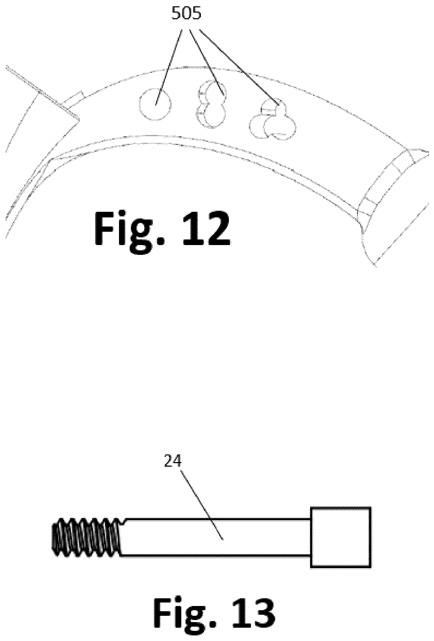

In the figures, particularly in FIG. 12, it can be seen that the handle **301,** the hand grip **501** and the handle-guide **104** each have series of orientation marks **505** which allow the surgeon to ascertain the angular orientation (anteversion) of the spherical component insertion device **300** or of the Kirschner wire centering device **100.** Said series of marks consist of objects (apertures, inserts, etc.) with a transparency to X-rays that is different from that of the rest of the instrument. Each object will have a different angle ("0°, 8° and 16°;" "0°, 10° and 20°;" "0°, 5°, 10° and 15°," etc.).

Thus, when the surgeon uses the image intensifier, the mark **505** that is not deformed will indicate the angle of anteversion applied relative to the femur. FIG. 12 has been produced with an angle corresponding to the first mark **505,** and therefore said mark is not deformed but the others are.

The handle **301,** the handle-guide **104** or the hand grip **501** may be identical or may even be the same element.

Once the anteversion has been selected, the diaphyseal nail **3** may be fastened to the femur by positioning the distal screws. In the lower portion of the diaphyseal nail **3** one or more distal screws are arranged in each fastening aperture **32.** This insertion may be by any known method of the prior art, whether through the lower incision or through the middle incision **IM,** depending on the position or orientation of the fastening apertures **32.**

Once this has been done, the diaphyseal nail insertion device **500** is withdrawn, leaving the hip arthroplasty device now placed in its final position.

The invention therefore comprises, in addition to the prosthesis, an entire series of preferred instruments which are designed to assist in its positioning and to remove the femoral head:
- a Kirschner wire centering device **100;**
- a cannulated femoral or humeral head extractor **200;**
- a spherical component insertion device **300;**
- a metaphyseal component insertion device **400;**
- a diaphyseal nail insertion device **500.**

It can be seen that the first two insertion devices **300, 400** serve as guides for the insertion of the next insertion device. In other words, the spherical component insertion device **300** serves as a guide for inserting the metaphyseal component insertion device **400,** and this in turn serves as a guide for inserting the diaphyseal nail **500** (FIG. 10), thus minimizing error and facilitating manipulation by the surgeon.

However, other different instruments may be used which would allow the prosthesis to be assembled at the site of use, but without facilitating the work of the surgeon, for example, always using straight insertion devices.

Moreover, the transmission of the load from the prosthesis to the femur is distributed at various points: the metaphyseal component **2** transmits its load to the calcar, whilst the diaphyseal nail **3** transmits the load to the diaphysis and to each distal screw through the corresponding fastening aperture **32.** All this provides sufficient stability and cementing is therefore not mandatory, which avoids the complications of cementing the hip arthroplasty.

However, if cementing is preferred, the metaphyseal component **2** and/or the diaphyseal nail **3** may have a channel to allow the introduction of cement from the outside, once in position, through a cannula, syringe, etc., in order to increase the contact between the different portions of the prosthesis and the femur.

All the materials must be biocompatible, particularly those that remain inside the patient's body.

## Claims

1. A prosthesis for replacing a femoral or humeral head, the prosthesis comprising:
- a spherical component (1) having a substantially hemispherical shape with a rear face (12) having a bore (13);
- a metaphyseal component (2) made up of a straight element with a transverse, approximately central, aperture (21), and a shank (22) configured to attach to the bore (13) of the head component (1), the metaphyseal component (2) including a longitudinal hole (23) which passes through the aperture (21), an opening of said hole being on a side of the metaphyseal component (2) opposite to the shank (22);
- a diaphyseal nail (3), intended for insertion in a femoral or humeral canal, having a first segment (31) and a second segment (33), wherein the second segment (33) of the diaphyseal nail (3) is configured to be arranged in the aperture (21) of the metaphyseal component (2); and
- a locking device (24) configured to be arranged in the longitudinal hole (23);
**characterized in that**:
- the rear face (12) of the spherical component (1) has recesses (14); and
- the second segment (33) of the diaphyseal nail (3) is configured to allow the locking device (24) to pass through the second segment (33).

2. Prosthesis according to claim 1, the metaphyseal component (2) of which has a grooving over its entire surface.

3. Prosthesis according to claim 1, the locking device (24) of which passes completely through the diaphyseal nail (3) and is threaded at its tip.

4. Prosthesis according to claim 1, of which the diaphyseal nail (3) has a bracket (34) in the second segment (33) configured to allow a portion of the metaphyseal component (2) on the side opposite the shank (22) to rest thereon.

5. A kit for arthroplasty comprising a prosthesis for replacing a femoral or humeral head according to claim 1, wherein one or more fastening apertures (32) are situated on the first segment (31) of the diaphyseal nail (3) for distal screws for fastening to the femur, the kit further comprising a set of tools for placement of the prosthesis.

6. Kit for arthroplasty, according to claim 5, wherein the set of tools comprises a cannulated extractor for the femoral or humeral head, that comprises a tubular part (201) with a grip (202) at one end and a conical or cylindrical drill bit (203) at the opposite end, the drill bit (203) having a passage at its tip configured to allow a Kirschner wire (102, 102') to pass tightly through, and the grip (202) having a passage opening aligned with an aperture in the tubular part (201).

7. Kit for arthroplasty, according to claim 6, wherein the drill bit (203) of the cannulated extractor is made up of various internally convergent and flexible elements, and an internal diameter of the tubular part (201) is greater than the passage made in the drill bit (203).

8. Kit for arthroplasty, according to claim 6, wherein the cannulated extractor has a holder (204) for controlling retractable claws (205) close to the drill bit (203).

9. Kit for arthroplasty, according to claim 8, whose holder (204) is a nut on a screw (206) inside the tubular part (201), which is cannulated for the passage of the Kirschner wire (102), connected to the retractable claws (205).

10. Kit for arthroplasty, according to claim 5, wherein the set of tools comprises a Kirschner wire centering device for positioning the prosthesis that has the general shape of an inverted 'U,' with:
- a centering nail (101) at one end, intended for insertion in the femoral or humeral canal, with a vertical opening (105) in its lower portion; and
- a handle-guide (104) at an opposite end with a vertical opening (105') in its lower portion, an upper edge of which is aligned with an upper edge of the vertical opening of the centering nail (101).

11. Kit for arthroplasty, according to claim 10, wherein the Kirschner wire centering device has a sleeve (103) in the vertical opening (105') of the handle-guide (104) with an upper passage for a first Kirschner wire (102), aligned with the upper edge of the vertical opening (105) of the centering nail (101).

12. Kit for arthroplasty, according to claim 11, whose sleeve (103) has another passage for a second Kirschner wire (102').

13. Kit for arthroplasty, according to claim 10, where the handle-guide (104) of the Kirschner wire centering device (100) has marks (505) with a transparency to X-rays that is different from the rest of the Kirschner wire centering device (100), with different development angles.

14. Kit for arthroplasty, according to claim 5, where the set of tools comprises a spherical component insertion device (300) for the spherical component (1) that has the shape of an inverted 'U,' with:
- a handle (301) with a recess (304) open at the lower edge, and with an upper edge oriented toward the bore (13) of the spherical component (1); and
- a support (302) that is compatible with the recesses (14) of the spherical component (1), and can be released using an actuator (303).

15. Kit for arthroplasty, according to claim 14, whose sleeve (301) has marks (505) with a transparency to X-rays that is different from the rest of the spherical component insertion device (300), with different development angles.

16. Kit for arthroplasty, according to claim 5, wherein the set of tools comprises a metaphyseal component insertion device (400), for the metaphyseal component (2), **characterized in that** it consists of a straight, hollow rod, with a clamp (403) for the metaphyseal component (2) at one end and a gripper (404) at the opposite end.

17. Kit for arthroplasty, according to claim 16, wherein the metaphyseal component insertion device comprises two tubular, coaxial bodies, and the clamp (403) is made up of protrusions (406) in the outer tubular body, of a bayonet closure for the corresponding clips of the metaphyseal component (2), and also comprises a thread (407) close to the gripper (404) for locking the relative movement between both tubular bodies.

18. Kit for arthroplasty, according to claim 5, wherein the set of tools comprises a diaphyseal nail insertion device (500) for the diaphyseal nail (3) that has an inverted 'U' shape, with a hand grip (501) with a groove (503) open towards the lower portion thereof at a first end and a removable connection for the diaphyseal nail (3) at an opposite end.

19. Kit for arthroplasty, according to claim 18, where the hand grip (501) of the diaphyseal nail insertion device (500) has marks (505) with a transparency to X-rays that is different from the rest of the centering device (500), with different development angles.

## Patentansprüche

1. Prothese zum Ersatz eines Femur- oder Humeruskopfes, wobei die Prothese Folgendes umfasst:
- ein sphärisches Element (1) mit
im Wesentlichen halbkugelförmiger Gestalt und einer Rückseite (12) mit einer Bohrung (13);
- ein metaphysäres Element (2), bestehend aus einem geradlinigen Abschnitt mit einer annähernd zentralen, querverlaufenden Öffnung (21) und einem Schaft (22), der so ausgestaltet ist, dass er mit der Bohrung (13) des Kopfelements (1) verbindbar ist, wobei das metaphysäre Element (2) ein Längsloch (23) aufweist, das durch die Öffnung (21) verläuft,
und wobei eine Öffnung dieses Längslochs an einer dem Schaft (22) gegenüberliegenden Seite des metaphysären Elements (2) angeordnet ist;
- einen diaphysären Nagel (3), vorgesehen zur Einführung in einen Femur- oder Humeruskanal, mit einem ersten Abschnitt (31),
sowie einem zweiten Abschnitt (33), wobei der zweite Abschnitt (33) des diaphysären Nagels (3) so ausgestaltet ist, dass er in der Öffnung (21) des metaphysären Elements (2) angeordnet werden kann; und
- einer Verriegelungseinrichtung (24), die zur Anordnung in dem Längsloch (23) vorgesehen ist;
**dadurch gekennzeichnet, dass**:
- die Rückseite (12) des sphärischen Elements (1) Vertiefungen (14) aufweist; und
- der zweite Abschnitt (33) des diaphysären Nagels (3) so ausgestaltet ist, dass die Verriegelungseinrichtung (24) durch den zweiten Abschnitt (33) hindurchgeführt werden kann.

2. Prothese nach Anspruch 1, deren metaphysäres Element (2) über seine gesamte Oberfläche eine Riffelung aufweist.

3. Prothese nach Anspruch 1, bei der die Verriegelungseinrichtung (24) vollständig durch den diaphysären Nagel (3) hindurchgeführt ist und an ihrer Spitze ein Gewinde aufweist.

4. Prothese nach Anspruch 1, bei der der diaphysäre Nagel (3) im zweiten Abschnitt (33) eine Halterung (34) aufweist, die so ausgestaltet ist, dass ein Bereich des metaphysären Elements (2) an der dem Schaft (22) gegenüberliegenden Seite darauf abstützbar ist.

5. Kit für die Arthroplastie, umfassend eine Prothese zum Ersatz eines Femur- oder Humeruskopfes gemäß Anspruch 1, wobei
sich am ersten Abschnitt (31) des diaphysären Nagels (3) eine oder mehrere Befestigungsöffnungen (32) für distale Schrauben zur Fixierung am Femur befinden, und wobei das Kit ferner einen Werkzeugsatz zur Implantation der Prothese umfasst.

6. Kit für die Arthroplastie nach Anspruch 5, wobei der Werkzeugsatz einen kanülierten Extraktor für den Femur- oder Humeruskopf umfasst, der ein rohrförmiges Element (201) mit einem Griff (202) an einem Ende und einem konischen oder zylindrischen Bohrer (203) am gegenüberliegenden Ende aufweist, wobei der Bohrer (203) an seiner Spitze eine Durchgangsöffnung aufweist, die so ausgestaltet ist, dass ein Kirschner-Draht (102, 102') straff hindurchführbar ist, und wobei der Griff (202) eine Durchlassöffnung aufweist, die mit einer Öffnung im rohrförmigen Element (201) fluchtet.

7. Kit für die Arthroplastie nach Anspruch 6, wobei der Bohrer (203) des kanülierten Extraktors aus mehreren flexiblen, nach innen konvergierenden Elementen besteht, und wobei der Innendurchmesser des rohrförmigen Elements (201) größer ist als die im Bohrer (203) ausgebildete Durchgangsöffnung.

8. Kit für die Arthroplastie nach Anspruch 6, wobei der kanülierte Extraktor einen Halter (204) zur Steuerung von einziehbaren Krallelementen (205) in der Nähe des Bohrers (203) aufweist.

9. Kit für die Arthroplastie nach Anspruch 8, wobei der Halter (204) als Mutter auf einer Schraube (206) ausgebildet ist, die sich innerhalb des rohrförmigen Elements (201) befindet, wobei das rohrförmige Element (201) kanüliert ist, um den Durchtritt des Kirschner-Drahts (102) zu ermöglichen, und wobei die Schraube mit den einziehbaren Krallelementen (205) verbunden ist.

10. Kit für die Arthroplastie nach Anspruch 5, wobei der Werkzeugsatz eine Zentriervorrichtung für einen Kirschner-Draht zur Positionierung der Prothese umfasst, die im Wesentlichen die Form eines umgekehrten "U" aufweist, mit:
- einem Zentriernagel (101) an einem Ende, vorgesehen zur Einführung in den Femur- oder Humeruskanal, mit einer vertikalen Öffnung (105) in seinem unteren Abschnitt; und
- einem Führungsgriff (104) am gegenüberliegenden Ende mit einer vertikalen Öffnung (105') in seinem unteren Abschnitt, wobei die obere Kante der Öffnung mit der oberen Kante der vertikalen Öffnung des Zentriernagels (101) fluchtet.

11. Kit für die Arthroplastie nach Anspruch 10, wobei
die Zentriervorrichtung für den Kirschner-Draht eine
Hülse (103) in der vertikalen Öffnung (105') des Führungsgriffs (104) aufweist, mit einer oberen Durchgangsöffnung für einen ersten Kirschner-Draht (102), wobei diese Durchgangsöffnung mit der oberen Kante der vertikalen Öffnung (105) des Zentriernagels (101) fluchtet.

12. Kit für die Arthroplastie nach Anspruch 11, wobei die Hülse (103) eine weitere Durchgangsöffnung für einen zweiten Kirschner-Draht (102') aufweist.

13. Kit für die Arthroplastie nach Anspruch 10, wobei der Führungsgriff (104) der Zentriervorrichtung (100) für den Kirschner-Draht Markierungen (505) aufweist, die gegenüber dem übrigen Teil der Zentriervorrichtung (100) eine abweichende Röntgentransparenz besitzen und unterschiedliche Anlegewinkel anzeigen.

14. Kit für die Arthroplastie nach Anspruch 5, wobei der Werkzeugsatz eine Einsetzvorrichtung (300) für das sphärische Element (1) umfasst, die im Wesentlichen die Form eines umgekehrten "U" aufweist, mit:
- einem Griff (301) mit einer an der Unterkante geöffneten Ausnehmung (304), deren Oberkante in Richtung der Bohrung (13) des sphärischen Elements (1) ausgerichtet ist; und
- einem Träger (302), der mit den Vertiefungen (14) des sphärischen Elements (1) kompatibel und mittels eines Betätigungsmechanismus (303) lösbar ausgeführt ist.

15. Kit für die Arthroplastie nach Anspruch 14, wobei der Griff (301) Markierungen (505) aufweist, die eine von der übrigen Einsetzvorrichtung (300) abweichende Röntgentransparenz besitzen und unterschiedliche Anlegewinkel anzeigen.

16. Kit für die Arthroplastie nach Anspruch 5, wobei der Werkzeugsatz eine Einsetzvorrichtung (400) für das metaphysäre Element (2) umfasst, **dadurch gekennzeichnet, dass** sie aus einer geradlinigen, hohlen Stange besteht, die an einem Ende eine Klemme (403) für das metaphysäre Element (2) und am gegenüberliegenden Ende einen Greifer (404) aufweist.

17. Kit für die Arthroplastie nach Anspruch 16, wobei die Einsetzvorrichtung für das metaphysäre Element zwei koaxiale, rohrförmige Körper umfasst, und wobei die Klemme (403) aus Vorsprüngen (406) im äußeren Rohrkörper besteht, die einen Bajonettverschluss für die entsprechenden Raststrukturen des metaphysären Elements (2) bilden, wobei die Einsetzvorrichtung ferner ein Gewinde (407) nahe dem Greifer (404) aufweist, um die Relativbewegung zwischen beiden Rohrkörpern zu arretieren.

18. Kit für die Arthroplastie nach Anspruch 5, wobei der Werkzeugsatz eine Einsetzvorrichtung (500) für den diaphysären Nagel (3) umfasst, die eine umgekehrte "U"-Form aufweist, mit einem Handgriff (501), der an einem ersten Ende eine Rille (503) aufweist, die zur Unterseite hin geöffnet ist, und an einem gegenüberliegenden Ende eine lösbare Verbindung für den diaphysären Nagel (3).

19. Kit für die Arthroplastie nach Anspruch 18, wobei der Handgriff (501) der Einsetzvorrichtung für den diaphysären Nagel Markierungen (505) aufweist, die eine von der übrigen Zentriervorrichtung (500) abweichende Röntgentransparenz aufweisen und unterschiedliche Anlegewinkel anzeigen.

## Revendications

1. Prothèse destinée à remplacer une tête fémorale ou humérale, la prothèse comprenant :
- un composant sphérique (1) ayant
une forme sensiblement hémisphérique avec une face arrière (12) comportant un alésage (13) ;
- un composant métaphysaire (2) constitué d'un élément droit avec une ouverture transversale (21), approximativement centrale, et une tige (22) configurée pour se fixer à l'alésage (13) du composant de tête (1), le composant métaphysaire (2) comprenant un trou longitudinal (23) traversant l'ouverture (21), une ouverture dudit trou étant située sur un côté du composant métaphysaire (2) opposé à la tige (22)
- un clou diaphysaire (3), destiné à être inséré dans un canal fémoral ou huméral, comprenant un premier segment (31)
et un second segment (33), ledit second segment (33) du clou diaphysaire (3) étant configuré pour être disposé dans l'ouverture (21) du composant métaphysaire (2) ; et
- un dispositif de verrouillage (24) configuré pour être disposé dans le trou longitudinal (23) ;
**caractérisée en ce que** :
- la face arrière (12) du composant sphérique (1) présente des évidements (14) ; et
- le second segment (33) du clou diaphysaire (3) est configuré pour permettre au dispositif de verrouillage (24) de traverser le second segment (33)

2. Prothèse selon la revendication 1, dans laquelle le composant métaphysaire (2) présente des rainures sur toute sa surface.

3. Prothèse selon la revendication 1, dans laquelle le dispositif de verrouillage (24) traverse entièrement le clou diaphysaire (3) et est fileté à son extrémité.

4. Prothèse selon la revendication 1, dans laquelle le clou diaphysaire (3) comprend un support (34) dans le second segment (33) configuré pour permettre à une partie du composant métaphysaire (2), du côté opposé à la tige (22), de reposer dessus.

5. Kit pour arthroplastie comprenant une prothèse selon la revendication 1, dans lequel une ou plusieurs ouvertures de fixation (32) sont situées sur le premier segment (31) du clou diaphysaire (3) pour des vis distales destinées à la fixation au fémur, le kit comprenant en outre un ensemble d'outils pour la mise en place de la prothèse.

6. Kit pour arthroplastie selon la revendication 5, dans lequel l'ensemble d'outils comprend un extracteur canulé pour la tête fémorale ou humérale, comprenant une partie tubulaire (201) avec une poignée (202) à une extrémité et une mèche conique ou cylindrique (203) à l'extrémité opposée, ladite mèche (203) comportant un passage à sa pointe configuré pour permettre le passage ajusté d'un fil de Kirschner (102, 102'), et la poignée (202) comportant une ouverture de passage alignée avec une ouverture dans la partie tubulaire (201).

7. Kit pour arthroplastie selon la revendication 6, dans lequel la mèche (203) de l'extracteur canulé est constituée de divers éléments souples et convergents vers l'intérieur, et un diamètre interne de la partie tubulaire (201) est supérieur au passage réalisé dans la mèche (203).

8. Kit pour arthroplastie selon la revendication 6, dans lequel l'extracteur canulé comprend un support (204) pour commander des griffes rétractables (205) à proximité de la mèche (203).

9. Kit pour arthroplastie selon la revendication 8, dans lequel le support (204) est un écrou monté sur une vis (206) à l'intérieur de la partie tubulaire (201), laquelle est canulée pour le passage du fil de Kirschner (102), reliée aux griffes rétractables (205).

10. Kit pour arthroplastie selon la revendication 5, dans lequel l'ensemble d'outils comprend un dispositif de centrage pour fil de Kirschner destiné au positionnement de la prothèse, ayant la forme générale d'un « U » inversé, avec :
- un clou de centrage (101) à une extrémité, destiné à être inséré dans le canal fémoral ou huméral, avec une ouverture verticale (105) dans sa partie inférieure ; et
- une poignée-guide (104) à l'extrémité opposée avec une ouverture verticale (105') dans sa partie inférieure, dont un bord supérieur est aligné avec un bord supérieur de l'ouverture verticale du clou de centrage (101).

11. Kit pour arthroplastie selon la revendication 10, dans lequel le dispositif de centrage pour fil de Kirschner comporte une douille (103) dans l'ouverture verticale (105') de la poignée-guide (104), avec un passage supérieur pour un premier fil de Kirschner (102), aligné avec le bord supérieur de l'ouverture verticale (105) du clou de centrage (101).

12. Kit pour arthroplastie selon la revendication 11, dans lequel la douille (103) comporte un autre passage pour un second fil de Kirschner (102').

13. Kit pour arthroplastie selon la revendication 10, dans lequel la poignée-guide (104) du dispositif de centrage pour fil de Kirschner (100) comporte des repères (505) ayant une transparence aux rayons X différente de celle du reste du dispositif (100), avec différents angles d'inclinaison.

14. Kit pour arthroplastie selon la revendication 5, dans lequel l'ensemble d'outils comprend un dispositif d'insertion du composant sphérique (300) pour le composant sphérique (1), ayant la forme d'un « U » inversé, avec :
- une poignée (301) avec un évidement (304) ouvert sur le bord inférieur, et avec un bord supérieur orienté vers l'alésage (13) du composant sphérique (1) ; et
- un support (302) compatible avec les évidements (14) du composant sphérique (1), pouvant être commandé à l'aide d'un actionneur (303).

15. Kit pour arthroplastie selon la revendication 14, dans lequel la poignée (301) comporte des repères (505) ayant une transparence aux rayons X différente de celle du reste du dispositif d'insertion (300), avec différents angles d'inclinaison.

16. Kit pour arthroplastie selon la revendication 5, dans lequel l'ensemble d'outils comprend un dispositif d'insertion du composant métaphysaire (400), pour le composant métaphysaire (2), **caractérisé en ce qu'**il est constitué d'une tige droite et creuse, avec une pince (403) pour le composant métaphysaire (2) à une extrémité et une poignée de préhension (404) à l'extrémité opposée.

17. Kit pour arthroplastie selon la revendication 16, dans lequel le dispositif d'insertion du composant métaphysaire comprend deux corps tubulaires coaxiaux, la pince (403) étant constituée de saillies (406) sur le corps tubulaire externe, d'une fermeture à baïonnette pour les clips correspondants du composant métaphysaire (2), et comprenant en outre un filetage (407) proche de la poignée de préhension (404) afin de verrouiller le mouvement relatif entre les deux corps tubulaires.

18. Kit pour arthroplastie selon la revendication 5, dans lequel l'ensemble d'outils comprend un dispositif d'insertion du clou diaphysaire (500) pour le clou diaphysaire (3), ayant la forme d'un « U » inversé, avec une poignée (501) comportant une rainure (503) ouverte vers le bas à une première extrémité et une connexion amovible pour le clou diaphysaire (3) à l'extrémité opposée.

19. Kit pour arthroplastie selon la revendication 18, dans lequel la poignée (501) du dispositif d'insertion du clou diaphysaire (500) comporte des repères (505) ayant une transparence aux rayons X différente de celle du reste du dispositif de centrage (500), avec différents angles d'orientation.
